# EUROPÄISCHE PATENTSCHRIFT

(11) **EP 1 294 386 B1**
(45) Veröffentlichungstag und Bekanntmachung des Hinweises auf die Patenterteilung: **22.11.2006**
(21) Anmeldenummer: 01962733.0
(22) Anmeldetag: 12.06.2001
(51) Int. Cl.: A61K 35/14, C07K 14/805, C08G 65/32

(54) **KÜNSTLICHE SAUERSTOFFTRÄGER AUS VERNETZTEM MODIFIZIERTEN HUMAN- ODER SCHWEINEHÄMOGLOBIN, VERFAHREN ZU IHRER HERSTELLUNG AUS GEREINIGTEM MATERIAL, SOWIE DEREN VERWENDUNG**
SYNTHETIC OXYGEN CARRIERS MADE FROM CROSS-LINKED MODIFIED HUMAN OR PORCINE HAEMOGLOBIN, METHOD FOR A PREPARATION THEREOF FROM PURIFIED MATERIAL AND USE THEREOF
TRANSPORTEUR D'OXYGENE ARTIFICIEL A BASE D'HEMOGLOBINE PORCINE OU HUMAINE MODIFIEE ET RETICULEE, PROCEDE POUR SA PRODUCTION A PARTIR D'UNE SUBSTANCE PURIFIEE ET SON UTILISATION

(30) Priorität: 29.06.2000 DE 10031740
(43) Veröffentlichungstag der Anmeldung: 26.03.2003
(73) Patentinhaber: SanguiBioTech GmbH, 58455 Witten an der Ruhr (DE)
(72) Erfinder: BARNIKOL, Wolfgang, 55128 Mainz (DE); BURKHARD, Oswald, D-67819 Kriegsfeld (DE); PÖTZSCHKE, Harald, 65191 Wiesbaden (DE); DOMACK, Ulrike, 55268 Nieder-Olm (DE); DINKELMANN, Stephanie, 67659 Kaiserslautern (DE); FIEDLER, Bernd, 59427 Unna (DE); MANZ, Birgit, D - 34131 Kassel (DE)
(74) Vertreter: Müller, Claudia
(86) Internationale Anmeldenummer: PCT/EP2001/006613
(87) Internationale Veröffentlichungsnummer: WO 2002/000230

(56) Entgegenhaltungen:
- US-A- 5 380 824
- DATABASE DERWENT [Online] XP002182224
- POETZSCHKE H ET AL: "Molar masses and structure in solution of haemoglobin hyper-polymers - a common calibration of size exclusion chromatography of these artificial oxygen carriers." ARTIFICIAL CELLS BLOOD SUBSTITUTES AND IMMOBILIZATION BIOTECHNOLOGY, Bd. 25, Nr. 6, 1997, Seiten 527-540, XP001033782 ISSN: 1073-1199
- HANEY CHAD R ET AL: "Purification and chemical modifications of hemoglobin in developing hemoglobin based oxygen carriers." ADVANCED DRUG DELIVERY REVIEWS., Bd. 40, Nr. 3, 28. Februar 2000 (2000-02-28), Seiten 153-169, XP001029599 ISSN: 0169-409X

## Beschreibung

Die vorliegende Erfindung umfaßt gemäss den Ansprüchen die Herstellung chemisch modifizierter, vernetzter Hämoglobine mit verbesserten funktionellen Eigenschaften, die nach diesem Verfahren hergestellten vernetzten Hämoglobine, sowie ihre Verwendung als künstliche Sauerstoffträger. Das Herstellungsverfahren ist gekennzeichnet sowohl durch technisch Einfachheit als auch hohe Ausbeuten.

Hochreines desoxygeniertes Hämoglobin wird unter dem Schutz eines Antioxidationsmittels mit einem Effektor der Sauerstoffbindung, insbesondere Pyridoxal-5-phopshat, kovalent konjugiert, danach erfolgt die Polymerisierung des Hämoglobins mit Glutardialdehyd bei sehr starker Zunahme des Volumens des Reaktionsgemisches und somit sehr starker Verdünnung der Reaktanden während der Zugabe des Vernetzers. Anschließend wird nach Verdünnung mit Wasser an die vernetzten Hämoglobine ein Polyethylenoxid-Derivat chemisch angeknüpft. Erhalten werden mit Blutplasma vertägliche Polymere mit optimierter Sauerstoffbindungs-Charakteristik, die als künstliche Sauerstoffträger, insbesondere aufgeteilt in einen nieder- und einen hochmolekularen Anteil als Blutsubstitut bzw. als Blutadditiv, beispielsweise bei der Behandlung von Sauerstoffmangelzuständen, Verwendung finden können.

In der Medizin ist es aus verschiedenen klinischen indikationen wunschenswert, ein künstliches Unterstützungssystem für den Sauerstofftranspoort verfügbar zu haben. Im Falle eines akuten Blutverlustes erscheint es nämlich nicht nur sinnvoll, das Flüssigkeitsvolumen isoton und isonkotisch zu ersetzen, sondern auch eine weitere wesentliche Funktion des Blutes, nämlich die des Sauerstofftransports zu restituieren. Bei sinkender Bereitschaft zur Blutspende stehen immer seltener im akuten Katastrophenfall (u. a. auch im Kriegsfall) passende Blutkonserven zur Verfügung, um insbesondere einen unvorhersehbaren Bedarf decken zu können. Die momentane Verfügbarkeit geeigneter Blutkonserven bringt zudem erhebliche logistische Probleme mit sich. Ferner lassen sich Blutkonserven in der Regel nur etwa 35 Tage lagern und müssen deshalb ständig erneuert werden - was erhebliche Kosten bereitet - während künstliche Lösungen wesentlich länger aufzubewahren sind, da sie gegebenenfalls eingefroren werden können. Abhängig von der Lagerzeit säuern sich Blutkonserven intrazellulär an, dadurch ist ihre Sauerstoffbindungscharakteristik akut keineswegs optimal, vielmehr muß diese im Organismus zunächst wieder regeneriert werden. Dagegen funktioniert ein künstlicher Sauerstoffträger vom ersten Moment an optimal. Der zunehmend mangelnden Bereitschaft zur Blutspende steht auf der anderen Seite die steigende Überalterung der Bevölkerung bedarfserhöhend gegenüber. Zugleich vermindert sich wegen der Überalterung auch die Zahl der potentiellen Blutspender. Ebenso ist wegen unübersehbarer Infektionsrisiken (Immunschwäche, Hepatitis) die Bevölkerung der Vorstädte ("slums") als Blutspender ausgefallen. Ein künstlicher Sauerstoff-transportierender Blutersatz wäre auch, unabhängig von der Blutgruppe, universal. Es ist zudem möglich, daß mit einem solchen Blutersatz ein Volumenmangelschock eher durchbrochen werden kann als mit einer Blutkonserve, da die Erythrozyten in der Konserve versteift sind und dadurch eine verringerte Kapillardurchgängigkeit aufweisen. Jedenfalls haben Tierversuche ergeben, daß ein Volumenmangelschock mit sauerstofftransportierendem Blutersatz wirksamer bekämpft werden kann, als mit einfachen Plasmaexpandern (Pabst R. (1977): "Sauerstofftransport mit stromafreien Hämoglobinlösungen und Fluorocarbonen", *Med. Klin.* 72: 1555 - 1562, Keipert P. E., Chang T. M. S. (1985): "Pyridoxylated Polyhemoglobin as a Red Cell Substitute for Resuscitation of Lethal Hemorrhagic Shock in Conscious Rats", *Biomater., Med. Dev., Artif. Organs* 13: 1 - 15). Weitere Anwendungen eines künstlichen Sauerstoffträgers kommen hinzu: Komplizierte operative Eingriffe, welche notwendig mit hohen Blutverlusten einhergehen, lassen sich zunehmend weniger durchführen, weil die entsprechenden Blutkonserven fehlen. Demgegenüber erarbeitet man immer größere und invasivere operative Eingriffe - wozu insbesondere auch Transplantationen zählen -, deren Ausführung im Einzelfall entscheidend von der Verfügbarkeit ausreichend vieler geeigneter Blutkonserven abhängt. Weiterhin sind für eine Transplantation vorgesehene Organe weit besser zu konservieren, wenn sie mit (künstlichen) Sauerstottträgern perfundiert werden. Allein eine Lebertransplantation benötigt bis zu 100 Transfusionseinheiten zu je 450 mL. In Fällen polytraumatischer Schädigungen (beispielsweise durch einen Autounfall) werden ähnliche große Mengen benötigt.
Aber nicht nur im Falle eines akuten Blutverlustes, sondern auch im Falle chronischer Durchblutungsstörungen (insbesondere cerebrale, koronare, renale und periphere - beispielsweise beim Hörsturz - oder einer anämischen Krise etwa im Falle chronischer Osteomyelitis oder nach Tumor-Chemotherapie) besteht ein Bedarf für einen künstlichen Sauerstoff-transportierenden Blutzusatz. Auch um im Falle eines fetalen Sauerstoffmangels durch eine Plazenta-Insuffizienz einen drohenden Abort zu verhindern oder zur Verhinderung von Sauerstoffmangelschäden unter der Geburt, bietet sich die Anwendung des Additivs an oder aber zur Entwöhnung von der Beatmung. Ein Bedarf solcher Art ist sogar wesentlich größer als für den vorher genannten Fall eines akuten Blutverlustes: Jährlich sind für etwa 750 000 Menschen in Deutschland solche chronischen Durchblutungsstörungen die Todesursache. Hinzu kommen noch die Krankheitsfälle aus dieser Ursache. An Krebs dagegen sterben jährlich lediglich etwa die Hälfte Menschen. Man versucht bekanntermaßen chronische Sauerstoffgewebsmängel durch hyperbare Sauerstoffzufuhr zu therapieren. Abgesehen davon, daß diese Therapie nur solange wirkt, wie der Sauerstoffüberdruck herrscht und abgesehen davon, daß das Vorgehen nicht ungefährlich ist, besteht hierbei die Gefahr oxidativer Gewebeschädigung durch radikalische Sauerstoffreaktionen, die über entsprechende Reaktionsprodukte nachweisbar sind. Ein künstlicher Sauerstoffträger wirkt, solange er vorhanden ist, und er offeriert dem Gewebe sogenannten Niederdruck-Sauerstoff, so dass die genannten Schäden nicht aufträten.
Die Anwendung eines sauerstofftransportierenden Blutadditivs als vorübergehende Unterstützung des endogenen Sauerstofftransportsystems stellt eine weitere Möglichkeit und Alternative zur Bekämpfung des chronischen geweblichen Sauerstoffmangels dar, den man bisher mit durchblutungsfördernden Mitteln(z.B. Gefäßdilatoren) zu therapieren versucht.
Für die Art der Anwendung kommt dem Konzept sehr zu gute, daß es sich um eine funktionelle Sauerstoff-Therapie handelt: Nicht das Substrat (der Sauerstoff) wird appliziert, sondern die Funktion des Trägersystems wird verbessert, das den Sauerstoff zu den Geweben bringt. Dies macht die Therapie in ihrer Wirkung multiplikativ, somit sehr effektiv, und gibt ihr gleichsam einen katalytischen Charakter. Hinzu kommen noch deutliche Hinweise, daß ein im Plasma gelöster Sauerstoffträger viel wirksamer ist, als ein in Erythrozyten "abgepackter".
Weiterhin kann ein solcher künstlicher Blutersatz frei von bekannten Erregern hergestellt werden; Infeküonsprobleme, wie Hepatitis und erworbener Immundefekt (AIDS) sind auf diese Weise vermeidbar.
Eine zusätzliche potentielle Empfängergruppe für künstliche sauerstofftransportierende Lösungen sind Patienten, welche eine allergische Reaktion, beispielsweise auf HLA-Antigene, erwarten lassen. Bisher versucht man die Leukozyten durch eine Filtration über Baumwolle aus Blutkonserven zu entfernen. Künstliche Blutersatzlösungen wären dagegen völlig frei von Leukozyten. In neuerer Zeit hat man an Schweinen beobachtet, dass nach Verbesserung der Sauerstoffversorgung (Herabsetzung der Sauerstoff-Affinität des Hämoglobins) das Schlagvolumen des Herzens verringert wird und die Herzfrequenz unbeeinflusst bleibt (Villereal M. C., et al. (1987): "Engineered Red Blood Cells with Modified Oxygen Transport Properties: A New Oxygen Carrier", *Biomater., Med. Dev., Artif. Organs* 15: 397). In einer anderen Arbeit (Bosman R. J., et al. (1992): "Free Polymerized Hemoglobin Versus Hyroxyethyl Starch in Resuscitation of hypovolemic Dogs", *Anesth. Analg.* 75: 811 - 817) wurde gezeigt, daß die Applikation sauerstofftransportierender Lösungen nach einem Volumenmangelschock beim Hund die Zunahme des Herzzeitvolumens verhindert und somit das Herz schont. Hier eröffnet sich die Möglichkeit, durch Verbesserung der Sauerstoffversorgung eine funktionelle kardiale Protektion zu erreichen, was beispielsweise im Falle eines Infarkts hilfreich ist. Das wäre ein völlig neuer Aspekt für die Anwendung sauerstofftransportierender Lösungen.
Eine weitere Anwendung solcher künstlicher Sauerstoffträger wäre die Erhöhung der Strahlungsempfindlichkeit von Tumoren, zumal sich mehr und mehr andeutet, daß molekulare, im Plasma gelöste Sauerstoffträger sehr viel effektiver Sauerstoff an das Gewebe abgeben, als Vollblut: Solche künstlichen Träger bewirken eine Synergie mit dem nativen (intraerythrozyteren) Träger. D. h., dass der molekular-disperse künstliche Träger im Blutplasma nicht nur *per se* am besten Sauerstoff aus der Kapillare abgibt, sondern zudem die Sauerstoffabgabe des vorhandenen nativen Systems verstärkt, und zwar über den Mechanismus der erleichterten Diffusion. Das bedeutet, daß man für diesen Zweck nur eine geringe Konzentration des künstlichen Trägers im Plasma benötigt. Trotzdem bleibt diese funktionelle Therapie (siehe oben) äußerst effektiv.
Alles Gesagte verdeutlicht den Bedarf eines künstlichen Sauerstofftransporteurs. Unerläßlich für die Nutzung eines künstlichen Sauerstoffträgers ist, dass sein Ausgangsmaterial in ausreichender Menge zur Verfügung steht. Verfallene Blutkonserven lösen das Problem somit nicht. Deshalb ist es notwendig, Tierhämoglobine zu verwenden, vorzugsweise von den wichtigsten Schlachttieren: Rind und/oder Schwein.
Aus der Darstellung des Bedarfs künstlichen Sauerstoffträger ergeben sich grob zwei Typen der Anwendung: einerseits im Falle eines starken Blutverlustes und andererseits im Falle eines chronischen Sauerstoffmangels. Im ersten Fall benötigt man zur Kompensation ein iso-onkotisches, Sauerstoff transportierendes Volumensubstitut (künstliche Sauerstoffträger der ersten Generation), im zweiten Fall dagegen ein Sauerstoff-transportierendes Blutadditiv (künstliche Sauerstoffträger einer zweiten und neuen Generation). Wie erwähnt, ist der letzte Fall der weitaus häufigere. Im übrigen erlaubt ein entsprechendes Blutadditiv, in Kombination mit einem sogenannte Plasmaexpander, auch die Therapie eines akuten Blutverlustes mit dem großen Vorteil, daß.der Arzt die Möglichkeit hat, sowohl die Gabe von Sauerstoffträgern als auch des Flüssigkeitsvolumen, auf den Bedarf des einzelnen Patienten abzustimmen
Auch der Organismus vermag beides (Menge des Sauerstoffträgers und Blutvolumen) unabhängig voneinander zu verändern, nämlich die Erythrozytenbildung über Erythropoetin und das Plasmavolumen über ein eigenes Regulationssystem. Beide Größen sind dadurch entkoppelt, daß der Träger im Blut einen sehr viel kleineren kolloidosmotischen Druck hat als das Plasma.

Bisher wurden von anderen drei grundsätzlich verschiedene Strategien zur Entwicklung eines künstlichen Sauerstoffträgers für das Blut verfolgt (Stand der Technik: Rudolph A. S. et al. (Hrsg.): *Red Blood Cell Substitutes: Basic Principles and Clinical Applications,* Marcel Dekker, New York u. a. 1998; Tsuchida E. (Hrsg.): *Blood Substitutes: Present and Future Perspectives,* Elsevier Science, Amsterdam 1998; Chang T. M. S. (Autor bzw. Hrsg.): *Blood Substitutes: Principles, Methods, Products and Clinical Trials, Volume 1* und ∼ *Volume* 2, Karger Landes, Basel u. a. 1997 und 1998)

Verwendung von Emulsionen mit Fluorkohlenwasserstoffen - neuerdings setzt man auch andere Halogene wie Brom ein -, in welchen Sauerstoff besonders gut löslich ist (Hirlinger W. K., et al. (1982): "Auswirkungen eines teilweisen Blutaustausches mit Fluosol DA 20% auf den intakten Organismus des Schweines", *Anästhesist* 31 660 - 666). Da die Fluokarbone lipophil sind, ist jedoch zu erwarten, daß Wechselwirkungen und Störungen in den Lipidschichten der Zellmembranen auftreten. Letztere sind integrierende funktionelle Bestandteile der Zelle. Zudem müssen die Fluokarbone mit Emulgatoren, wie Phospholipide dispergiert werden, welche zusätzlich mit den Membranen der Zellen interferieren können (so benannte künstliche Sauerstoffträger auf Basis von Fluorkarbonen der ersten Generation).
Eine weitere Strategie stellt die Mikroverkapselung hochkonzentrierter Lösungen natürlichen und auch chemisch modifizierten Hämoglobins in Phospholipidvesikeln unter Zusatz geeigneter Effektoren der Hämoglobinbindung ("künstliche Erythrozyten oder Hämosomen") dar (Ogata Y. (1994): "Characteristics of Neo Red Cells, Their Function and Safety: In-Vivo Studies", *Artificial Cells, Blood Substitutes, and lmmobilization Biotechnologies* 22: 875 - 881). Auf diesem Felde sind auch erste Tierversuche gelungen (Hunt C. A., et al. (1985): "Synthesis and Evaluation of a Protypal Artificial Red Cell", *Science* 230: 1165 - 1168). Die Vesikel hatten einen Durchmesser von weniger als 0,05 µm und waren damit vom Volumen her um gut zwei Zehnerpotenzen kleiner als natürliche rote Blutzellen.
Die dritte Strategie besteht in der Herstellung infundierbarer Hämoglobinlösungen. Der künstliche Sauerstoffträger liegt dann im Blut extrazellulär vor. Während man die beiden ersten Problemlösungen als Herstellen eines künstlichen Blutes ansehen kann, so daß die Schwierigkeit einer kolloid-osmotischen Interferenz nicht auftreten kann, stand bei dieser Problemlösung ein Plasmaexpander am Anfang, dessen Makromoleküle auch Sauerstoff transportieren können (so benannte künstliche Sauerstoffträger auf der Basis von Hämoglobin der ersten Generation).
Natives Hämoglobin ist hierfür nicht brauchbar, da es beispielsweise zu schnell über die Niere ausgeschieden wird. Eine chemische Modifikation ist deshalb unerläßlich. Im Rahmen dieser Strategie hat man beispielsweise das Hämoglobin über seine Aminogruppen kovalent an Dextrane gebunden oder selbst bis zu einem Molekulargewicht von etwa 700 000 g/mol polymerisiert. Ersteres wurde beispielsweise von der Firma Fresenius (Fresenius E. (1976): "Blood and Plasma Substitute - Comprising a Colloidal Solution of Hydroxyethyl Starch Coupled to Haemoglobin Free Stroma", Patentschrift DE-P 2616-086) und letzteres von der Firma Biotest (Bonhard K., et al. (1983): "Verfahren zur Gewinnung von hepatitissicheren, sterilen, pyrogenfreien und stromafreien Hämoglobinlösungen", Patentschrift DE-O 31 30 770) und der Firma Alza (Bonsen P. (1976): "Water-soluble Polymerized Hemoglobin", Patentschrift DE-O 26 07 706) verfolgt. Eine weitere Strategie bezüglich extrazellulärer Lösungen ist, das Hämoglobin zu stabilisieren, indem man es intratetramer vernetzt oder indem man Seitengruppen anhängt (Oligo-Ethylenglykol), ohne das (tetramere) Molekulargewicht wesentlich zu erhöhen (stabilisierte Hämoglobine (Matsushita M., et al. (1987): "In vivo Evaluation of Pyridoxylated Hemoglobin-Polyoxyethylene Conjugate", *Biomat., Artif. Cells, Artif. Org.* 15: 377). Wie weiter oben erwähnt, haben extrazelluläre Blutersatzlösungen in Tierversuchen bezüglich einer Schockbehandlung erfolgversprechende Ergebnisse gebracht.
Vorteil der Verwendung von Hämoglobinen als künstliche Sauerstoffträger - gegenüber Fluorkarbonen - ist, daß sich die günstigen Eigenschaften der natürlichen Sauerstoffbindung nutzen lassen. Dazu gehören die optimal angepaßte mittlere Sauerstoffaffinität, die homotrope Kooperativität, also die S-Form der Sauerstoffbindungskurve, sowie der (alkalische) Bohreffekt, der die Basis eines natürlichen selbstregulatorischen Mechanismus zur gezielten Sauerstoffabgabe an mangelversorgte Gewebe bildet.
Aus der diesbezüglichen Literatur geht klar hervor, daß eine intra-tetramere kovalente Verknüpfung der Hämoglobinuntereinheiten (Keipert P.E., et al. (1989): "Metabolism, Distribution, and Excretion of HbXL: A Nondissociation Interdimerically Crosslinked Hemoglobin with Exceptional Oxygen Offloading Capability", - in: Chang T. M. S., Geyer R. P. (Eds.): *Blood Substitutes,* Marcel Dekker, New York 1989) und/oder eine Polymerisation des Hämoglobins zu einer starken Erhöhung der Verweildauer im Blut führt (Chang T. M. S.(1987): "Modified Hemoglobin as Red Cell Blood Substitutes", *Biomater., Med. Devices Artif. Organs* 14: 323 - 328; Friedman H. J., et al. (1984): "In Vivo Evaluation of Pyridoxylated-Polymerized Hemoglobin Solution", *Surg., Gynecol., Obstet.* 159: 429 - 435). Dies ist eine wesentliche Voraussetzung für die klinische Brauchbarkeit solcher Lösungen.

Im Fall der extrazellulären molekular-dispersen künstlichen Sauerstoffträger hat man jedoch bisher an einem sehr großen Bedarfsfeld - dem chronischen Sauerstoffmangel - vorbeientwickelt, indem isonkotische Lösungen angestrebt wurden. Die, wie bereits erwähnt, wesentlich häufiger auftretenden Folgen chronischer Durchblutungsstörungen lassen sich jedoch nur durch sauerstofftransportierende Lösungen bessern, deren kolloidosmotischer Druck gegen den normalen (35 mbar) zu vernachlässigen ist, also nur mit Hilfe eines sauerstofftransportierenden Blutadditivs, gewissermaßen eines molekularen Erythrozytenkonzentrats". Dies sind künstliche Sauerstoffträger auf der Basis von Hämoglobin einer zweiten Generation.

Bei den verschiedenen Ansätzen, einen künstlichen Sauerstofftransporteur zu entwickeln, traten nachfolgend genannte Probleme auf:
- Erhöhung der Sauerstoff-Hämoglobin-Affinität: der Halbsättigungsdruck (P50) nimmt durch die chemische Modifikation am Hämoglobinmolekül ab. Dadurch wird die Abgabe des Sauerstoffs an das Gewebe erschwert. Dies tritt ausgeprägt bei der Bindung des Hämoglobins an Dextran auf. Um die Erhöhung der Sauerstoffaffinität zu vermeiden, hat man geeignete Effektoren (beispielsweise Pyridoxalphosphat) an die prosthetische Gruppe des Hämoglobins gebunden.
- Oftmals verkleinert sich zugleich der sogenannte n50-Wert (HILL-Index) als Ausdruck verringerter homotroper Kooperativität (abgeschwächte S-Förmigkeit der Sauerstoff-Hämoglobin-Bindungskurve), was ebenfalls die Versorgung der Gewebe mit Sauerstoff erschwert. Diese S-Förmigkeit der Sauerstoff-Hämoglobin-Bindungskurve erleichtert zugleich die Aufnahme des Sauerstoffs in der Lunge und dessen Abgabe an die Zellen. Fluorkarbone besitzen dagegen eine lineare "Sauerstoffbindungskurve" und haben daher nicht diesen funktionellen Vorteil.
- Der künstliche Sauerstoffträger hat oftmals eine zu geringe Verweildauer im Organismus, die Ausscheidung der gelösten Hämoglobine erfolgt über die Niere. Im Falle extrazellulärer Hämoglobinlösungen als künstliche Sauerstoffträger hat man versucht, die Ausscheidung durch intermolekulare Vernetzung zu verhindern; jedoch bleibt trotzdem die Verweildauer der extrazellulären Hämoglobine kleiner als gewünscht. Hämosomen werden dagegen durch das Retikuloendotheliale System des Organismus aus dem Plasma entfernt. Beispielsweise betrug die Halbwertszeit der künstlichen Erythrozyten (siehe oben) nur 5,8 Stunden.
- Zu großer kolloidosmotischer Druck: Dadurch kann es zum Volumenverlust kommen (Volumenmangelschock). Dieser Effekt tritt auf, wenn das Molekulargewicht des künstlichen Sauerstoffträgers mit dem der Plasmaproteine vergleichbar ist. Auch ist man hierdurch mit der Dosierung des künstlichen Sauerstoffträgers nicht frei, sondern muß auf die onkotischen Verhältnisse Rücksicht nehmen.
- Das onkotische Milieu des Plasmas wird weiterhin entscheidend durch den sogenannte zweiten Virialkoeffizienten (A₂-Wert) bestimmt: Dieser charakterisiert die Wechselwirkung des (stets makromolekularen) Sauerstoffträgers mit dem Lösungsmittel (Wasser). Die Synthese ist so einzurichten, daß dieser Wert nahe null ist.
- Zu hohe Viskosität der Trägerlösung: Diese geht in der Regel mit einem zu großen A₂-Wert einher, und sie tritt bevorzugt auf, wenn ein Träger aus Kettenmolekülen besteht. Eine zu große Viskosität tritt dem EINSTEINschen Viskositätsgesetz gemäß nicht auf, wenn die polymeren Trägermoleküle kugelig und kompakt sind.
- *In vitro -* Stabilität der Trägermoleküle. Diese bezieht sich einerseits auf den Zerfall der Moleküle und andererseits auf die oxidative Bildung von Met-Hämoglobin, welches keinen Sauerstoff mehr zu binden vermag und schließlich auf die Viskosität durch sich langsam ändernde Wechselwirkungen zwischen dem Träger und dem Albumin des Plasmas.
- Übermäßige Reaktion des retikuloendothelialen Systems (RES): Hauptsächlicher Einflußfaktor ist die molekulare Größe des künstlichen Trägers. Es gibt dafür eine kritische Grenze bei rund 0,3 µm: Größere Teilchen aktivieren das RES.
- Nieren- und Leberschädigung: Ein Nierenschock tritt vor allem dann auf, wenn stromahaltige Hämoglobinlösung verwendet wurde. Seit man die Lösungen ultrafiltriert, wurde eine Nierenschädigung nicht mehr beobachtet. Leberschädigungen wurden mit Hilfe des Plasmatransaminase-Spiegels indiziert, sie beruhen vermutlich auf zellulären Membran-Wechselwirkungen: die Leber besitzt eine offene Strombahn (fenestrierte Kapillaren).
- Zu überprüfen ist auch die Blutstillung; es sind Störungen im Sinne einer Förderung und einer Hemmung denkbar, zu achten ist insbesondere auf die Thrombozytenaggregation.
- Antigene Wirkung: Dazu wurde neuerdings in Homologversuchen an Ratten gezeigt, daß natives Hämoglobin nicht antigen wirksam ist und daß die Polymerisation mit Glutardialdehyd die Antigenität nicht erhöht (Hertzman C. M., et al. (1986): "Serum Antibody Titers in Rats Receiving Repeated Small Subcutaneous Injection of Hemoglobin or Polyhemoglobin. A Preliminary Report", *Int. J. Artif. Organs* 9: 179 - 182). In der gleichen Arbeit wird gezeigt, daß natives und polymerisiertes Human-Hämoglobin bei Ratten wenig antigen wirkt und daß der Effekt durch die Polymerisation höchstens geringgradig verstärkt wird.
- Toxische Wirkungen lassen sich als pyrogen, vasokonstriktorisch - beispielsweise an den Koronargefäßen - und endotoxisch differenzieren. Die vasokonstriktorische Wirkung beruht vermutlich auf dem Einfangen der Stickstoffmonoxid-Radikale, welche bekanntlich endogene vasodilatorische Steuersubstanzen sind; die vasokonstriktorische Wirkung lässt sich jedoch auch durch die hohe Wirksamkeit der künstlichenTräger erklären, indem die glatte Muskulatur "zu gut" mit Sauerstoff versorgt wird.
- *in vivo -* Stabilität: Zu denken ist an einen endogenen enzymatischen Abbau, beispielsweise durch Proteasen.
- Überlastung des Organismus mit Lipoiden (Emulgatoren): Diese Komplikation tritt bei der Anwendung mikroverkapselter Hämoglobinlösungen und Fluokarbonen auf; hierbei wurde an Ratten eine Komplementaktivierung über den alternativen Weg sowie eine Antikörperbildung festgestellt.
- Verträglichkeit der künstlichen Träger mit frischem menschlichen Blutplasma, abhängig vom pH-Wert kann es zu Fällungen kommen.
Vermutlich wegen der hier angeführten Probleme steht bislang ein künstlicher Sauerstoffträger für die klinische Routineanwendung nicht zur Verfügung.
Aus der Nennung der manigfachen Probleme geht hervor, dass es nach wie vor einen großen Anforderungskatalog an einen brauchbaren künstlichen Sauerstoffträger gibt.

In der Natur wird Sauerstoff stets in mikroskopischen Riesenaggregaten transportiert. Es haben sich hier zwei grundsätzlich verschiedene "Problemlösungen" entwickelt. Bei höheren Tieren (und auch beim Menschen) ist der molekulare Sauerstoffträger in Zellen (in den Erythrozyten) abgepackt. Zweitens entwickelten sich Sauerstoff bindende Riesenmoleküle, die sich nicht intrazellulär, sondern extrazellulär in einer Hämolymphe gelöst finden, diese Variante trifft man vorwiegend bei niederen Tieren in verschiedener Ausprägung an. Anneliden besitzen beispielsweise hochmolekulare Hämoglobine mit einem mittleren Molekular gewicht um 3 000 000 g/Mol als Sauerstoffträger. Ferner gibt es sogenannte Hämerythrine, bei welchen das Eisen, als Sauerstoffbinder, selbst direkt mit dem Protein molekular verknüpft ist. Schließlich findet man die Hämocyanine mit einem Molekulargewicht um 8 000 000 g/Mol, bei welchen Kupfer das Sauerstoff bindende Schwermetall-lon ist, vgl. auch Barnikol W. K. R., et al. (1996): "Hyperpolymere Hämoglobine als künstliche Sauerstoffträger. Ein innovativer Ansatz der medizinischen Entwicklung", *Therapiewoche* 46: 811 - 815.
Der entwicklungsgeschichtliche Übergang vom extrazellulär gelösten Sauerstoffträger zum Erythrozyten hat dazu geführt, daß sich der Sauerstoffgehalt der Blutflüssigkeit um den Faktor drei erhöht: Während 1 mL "Blut" des Regenwurms nur 3,6 µmol Sauerstoff maximal zu binden vermag, werden in dem gleichen Volumen des Menschenbluts bis zu 9,0 µmol Sauerstoff gebunden.

Der Regenwurm besitzt in seinem Blut Riesenmoleküle (Erythrocruorin) als Sauerstoffträger mit einem Molekulargewicht von etwa 3400000 g/mol mit rund 200 Bindungsstellen für Sauerstoff. Zudem ist das Molekül sehr kompakt und seine Quartärstruktur hochgeordnet. Das Molekül ist so groß, daß man es mit Hilfe des Elektronenmikroskops direkt sichtbar machen kann. Seine Konzentration in der Hämolymphe beträgt mindestens 6 g/dL. Vermessen der Sauerstoffbindungskurve unter simulierten In-vivo-Bedingungen ergibt einen Halbsättigungsdruck (P50) von 9,1 Torr (Barnikol W. K. R., Burkhard O. (1987): "Highly Polymerized Human Haemoglobin as an Oxygen Carrying Blood Substitute", *Advances in Experimental Medicine and Biology Volume 215:* 129 - 134; Barnikol W.K.R. (1986): "The Influence of Glutardialdehyde on the Oxygen Cooperativity of Human Hemoglobin", *Pflügers Archiv* 406: R 61). Dieses System versorgt also die Zellen des Regenwurms ausreichend mit Sauerstoff. Durch das extrem hohe Molekulargewicht hat das Hämoglobin des Regenwurms praktisch keine kolloidosmotische Wirkung mehr (nur etwa 0,4 mbar). Damit sind, wie im Blut des Säugers - der onkotische Druck der Erythrozyten beträgt nur 10⁻⁷ Torr - die beiden Funktionen Kolloidosmolarität und Sauerstoffbindung entkoppelt, und beide können als Stellgrößen vom Organismus frei variiert werden.
Will man das Prinzip desRegenwurm-Systems auf künstliche Sauerstoffträger, die auf menschlichem oder tierischem Hämoglobin beruhen, übertragen, so muß das Hämoglobin-Molekül so verändert werden, daß es als extrazelluläres Riesenmolekül mit vernachlässigbarem onkotischen Druck den normalen Sauerstofftransport der Erythrozyten zumindest zeitweilig unterstützen kann. Solche künstlichen Sauerstoffträger sind dann hochpolymerisiertes Hämoglobin, welches allen oben erwähnten Problematiken Rechnung tragen muß. Regenwurm-"Hämoglobin", welches zwar die Anforderungen an onkotischen Druck erfüllt, wird sich zu diesem Zweck für den Menschen nicht verwenden lassen, weil es eine vermutlich zu große Antigenität besitzt; zudem ist der Halbsättigungsdruck mit 9 Torr zu niedrig (Barnikol W. K. R., Burkhard O. (1987): "Highly Polymerized Human Haemoglobin as an Oxygen Carrying Blood Substitute", *Advances in Experimental Medicine and Biology, Volume 215:* 129 - 134; Barnikol W.K.R. (1986): "The Influence of Glutardialdehyde on the Oxygen Cooperativity of Human Hemoglobin", *Pflügers Archiv* 406: R 61). Zudem ist es vermutlich nicht in den erforderlichen Mengen gewinnbar.

Bisher wurde bei der Entwicklung künnstlicher Sauerstoffträger deren Halbsättigungdruck genau auf den für den Menschen normalen Wert von etwa 26 Torr einzustellen versucht. Tierversuche zeigen allerdings, daß ein molekulardisperser künstlicher Sauerstoffträger mit einem Halbsättigungsdruck von etwa 15 Torr Organe am besten oxygeniert (Conover et al. (1999), *Art. Cells, Blood Subst., and Immobil. Biotech.* 27 93 - 107). Andererseits zeigen aber Tierversuche auch, daß für eine ausreichende Sauerstoffversorgung mindestens ebenso wichtig eine genügend große Sauerstoffkapazität des Blutes ist (Moss G. S., et al. (1984): "Hemoglobin Solution - From Tetramer to Polymer", - in: *The Red Cell: Sixth Aven Arbor Conference,* Alan R. Riss, New York 1984: 191 - 210). Diese hängt wiederum von der möglichen Konzentration des Sauerstoffträgers im Plasma ab. Weiterhin zeigte sich hierbei, daß ein Halbsättigungsdruck von 26 Torr nicht unbedingt eingehalten werden muß. Wesentlich ist vielmehr, daß ein bestimmter kritischer Wert nicht unterschritten werdem darf.

Zu den Forderungen bei der Entwicklung eines künstlichen Sauerstoffsträgers kommt hinzu, dass ein Herstellungsverfahren möglichst einfach und damit wirtschaftlich sein soll, vor allem, da *ab initio* steril gearbeitet werden muss. Das Produkt soll in möglichst großer Ausbeute erhalten werden können. Es sollte zugleich Material zur Verwendung als Blut-Additiv sowie solches zur Verwendung als Sauerstoff-transportierende Blutvolumen-Substitut während der Vernetzungsreaktion entstehen.
Insbesondere ist ferner bei der Herstellung der künstlichen Polymeren aus Hämoglobin durch Verknüpfung der Hämoglobin-Moleküle über ihre Aminogruppen mittels geeigneter bifunktioneller Vernetzer darauf zu achten, dass sich nicht molekulare Netzwerke bilden, die unlöslich sind und daher die Produktausbeute verringern. Daher ist das Entstehen der sogenannten sogenannte Perkolations-Verteilung des Molekulargewichts zu verhindern:

Der vorliegenden Erfindung lag die Aufgabe zugrunde, hypo-onkotische künstliche Sauerstoffträger aus vernetztem Hämoglobin, die optimierte gute funktionelle Eigenschaften, insbesondere der Charakteristik der Sauerstoffbindung, besitzen und die geeignet sind als Pharmazeutikum im Menschen angewendet zu werden, in einem technisch einfachen Prozess in großer Ausbeute herstellen zu können.

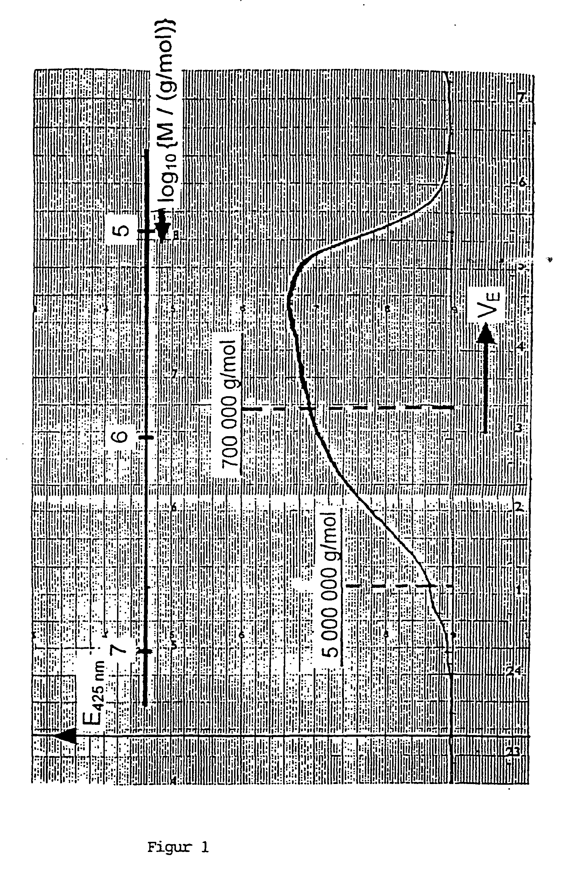

Die Aufgabe wird erfindungsgemäß, wie nachfolgend beschrieben, gelöst. Das fundamentale Problem der Entstehung einer Perkolationsverteilung der Multimerisationsgrade und Molekulargewichte durch die Vernetzung der polyfunktionellen Hämoglobine mit dem bifunktionellen Vernetzer Glutardialdehyd konnte überraschenderweise dadurch beseitigt werden, indem während der Vernetzung das Volumen des Reaktionsgemisches sehr stark (insgesamt 2- bis 10-fach) vergrößert wird, und zwar, indem zunächst zugleich der Vernetzer in verdünnter Lösung zugegeben und anschließend zusätzlich mit Wasser verdünnt wird. Dadurch entstehen vernetzte Hämoglobine mit hohem Vernetzungsgrad in großer Ausbeute, ohne dass eine Perkolationsverteilung der Molekulargewichte unter Bildung unlöslicher molekularer Netzwerke entsteht. Das Rohprodukt der Vernetzung zeichnet sich vielmehr auren eine bestimmte gewünschte (ungefähre) Obergrenze des Molekulargewichtsbereichs aus. Man erhält im Gel-Chromatogramm eine sogenannte Viereck-Verteilung des Molekulargewichtes (vgl. Fig. 1, Fig. 2 und Fig. 3).

Die erfindungsgemäße Herstellung umfasst also folgende Schritte:

In einer Eintopf-Reaktion wird Hämoglobin
i) zunächst desoxygeniert, insbesondere durch Überströmen mit Stickstoff;
ii) anschließend kovalent mit einem chemisch reaktiven Effektor der Sauerstoffbindung umgesetzt ;
iii) dann die Lösung mit einem chemisch nicht reaktiven Effektor versetzt und sodann
iv) das Hämoglobin mit Glutardialdehyd, unter sehr starker Verdünnung (2- bis 5-fach) des Volumens des Reaktionsgemisches, bei gleichzeitiger Zugabe des Vernetzters (in Lösung)), stabil kovalent miteinander vernetzt und anschließend das Reaktionsvolumen mit Wasser weiter erhöht (Gesamtverdünnung der Lösung auf das 2-10, insbesondere 2-7, besonders 5-6-fache an Volumen) und sodann
v) ein Polyethylenoxid kovalent angeknüpft.
Das erhaltene Produkt kann in bekannter Weise aufgearbeitet werden.

Bevorzugt stammt das Ausgangshämoglobin vom Schwein oder vom Menschen, ganz besonders bevorzugt vom Schwein, besonders vom Hausschwein.

Insbesondere wird erfindungsgemäß in Schritt iii) Glutardialdehyd in einer sehr stark verdünnten Lösung zeitgesteuert zugegeben. Bevorzugt erfolgt anschließend eine weitere Verdünnung und Erhöhung des Reaktionsvolumens mit Wasser, so dass die obengenannte Gesamtverdünnung erzielt wird.

Dabei ist bevorzugt, dass in Schritt iv) Glutardialdehyd in einer molaren Menge von 6-10, insbesondere 7-9 mol/mol, bezogen auf monomeres Hämoglobin, gelöst in 1-4, bevorzugt 1-2, insbesondere 1,5-2, ganz besonders bevorzugt 1,7-1,9 L Wasser, pro Liter ursprünglicher Reaktionslösung zugesetzt wird.

Dieser Zusatz erfolgt zeitgesteuert zwischen etwa 3 und 15, bevorzugt 3-10, insbesondere 4-6 Minuten.
Anschließend reagiert die Lösung zwischen 1 und 6 Stunden.

Es ist ferner bevorzugt, dass der das Hämoglobin enthaltenden Lösung vor der Umsetzung gemäss dem Schritt ii) 2-8, insbesondere 3-6, ganz besonders bevorzugt 3-4 Mol Natriumascorbat pro Mol unvernetztes Hämoglobin, zugegeben wird. Diese Umsetzung erfolgt über 0,5-6 Stunden, insbesondere 70-120 Minuten.

Ferner wird bevorzugt in Schritt ii) als Effektor Pyridoxal-5'-phosphat in einem molaren Verhältnis, bezogen auf monomeres Hämoglobin, von 0,5 bis 3, bevorzugt 1 bis 2,5 mol/mol kovalent über einen Zeitraum von 0,5-20, insbesondere 1-7 Stunden angebunden..

Erfindungsgemäß weiterhin vorteilhaft und bevorzugt ist es, dass in Schritt ii) sowie in Schritt iv) nach der kovalenten Anbindung von Pyridoxal-5'-phosphat sowie von Glutardialdehyd jeweils reduktives Natriumborhydrid hinzugesetzt wird.

Dies wird insbesondere in Schritt ii) in einer Menge von 1-9, bevorzugt 1-5, insbesondere 1-2,5 mol/mol z.B. für 30 bis 90 min., und in Schritt iv) in einer Menge von 5-20, insbesondere 6-12 mol/mol, jeweils bezogen auf monomeres Hämoglobin, für 15 bis 100 min. zugesetzt.

Besonders bevorzugt wird in Schritt iii) als chemisch nicht reaktiver Effektor 2,3-Bisphosphoglyzerat in einer Menge von 0,5-6, insbesondere 1-4 mol/mol, bezogen auf monomeres Hämoglobin, zugesetzt und etwa 5-50 Minuten, insbesondere 10-20 und ganz besonders 15 Minuten danach Glutardialdehyd zugesetzt.

Als Polyethylenoxid wird bevorzugt ein Polyethylenglykol-Ether z.B. mit einem C1-C5-Alkylrest wie Methyl, Ethyl Butyl, mit einem Molekulargewicht von 500 bis 3000 g/Mol angeknüpft, insbesondere ein Methoxy-Polyethylenglykol-Derivat mit einem Molekulargewicht von 1500-2500 g/Mol, insbesondere 2000 g/mol, wie insbesondere Methoxy-Polyethylenglykol-Succinimidyl-propionat-, in Mengen von 2-12, insbesondere 3-8 mol/mol Hämoglobin. Andere Derivatisierungsprodukte sind Methoxy-Polyethylenglykol-Succinimidyl-Succinamid und Methoxy-Polyethylenglykol-Succinimidyl-Oxyacetat.
Die Anknüpfung von Polyalkylenoxid an nicht vernetzte Hämoglobine ist beschrieben in US A-4 179 337, US 5 478 805, US 5 386 014, EP-A 0 206 448, EP-A 0 067 029, DE-OS 3 026 398.

Die Umsetzung erfolgt vorzugsweise über 1-4, insbesondere 2-3Stunden.

Es ist ferner vorteilhaft, dass alle Herstellungsreaktionen in insbesondere durch Tonometrie mit sauerstofffreien Gasen vom Sauerstoff befreiten Lösungen erfolgen. Das verwendete Verfahren ist beschrieben in: Pötzschke H.: Hyperpolymere des menschlichen Hämoglobins: Entwicklung präparativer Verfahren zu ihrer Synthese, Validierung analytischer Methoden und Geräte zu ihrer Charakterisierung, Dissertation, Medizinische Fakultät, Universität Wien, 1997.

Das erhaltene Produkt kann auf übliche Weise, wie nachfolgend geschildert, aufgearbeitet werden. Es weist insbesondere eine Verteilung der Molekulargewichte von 50 000 bis zu 5 000 000 auf, gegebenenfalls bis zu 10 000 000 g/Mol oder mehr.

Bevorzugt kann das erhaltene Produkt durch eine präparative stofftrennende Methode, wie beispielsweise eine präparative Volumenausschluss-Chromatographie, eine Ultrafiltration, eine fraktionierte Fällung, z.B. mit Polyalkylenoxiden oder Salzen wie Ammoniumsulfat als Fällmittel, oder eine Feld-Fluss-Fraktionier-Methode (vgl. Curling J.M. (Hrsg.) Methods of Plasma Protein Fractionation, Academic Press, London u.a. 1980, sowie die Patentschriften EP-A 0 854 151 und EP-A 95 107 280) in eine Fraktion mit großer und eine Fraktion mit niederer mittlerer molekularer Masse getrennt werden, wobei bevorzugt die Trenngrenze bei 700 000 g/mol liegt.

Aus dem Produkt mit hochmolekularer Masse und aus dem Produkt mit niedermolekularer Masse kann je eine pharmazeutische Zubereitung hergestellt werden, wobei aus dem niedermolekularen Anteil der Polymeren ein parenterales Blutsubstitut und aus dem höhermolekularen Anteil der Polymeren ein parenterales Blutadditiv erhalten wird.

Die pH-Wert-Einstellung vor (und nach) den einzelnen Reaktionsschritent erfolgt vorzugsweise mit Milchsäure oder Natronlauge auf Werte zwischen 6 und 10, je nach Reaktionsschritt, z.B. 6,5-7,5 vor Schritt ii), anschließend auf 7,5-8,5 und nachfolgend wieder auf 6,5-7,5 vor Schritt iii), danach auf 7,5 bis 9, sowie vor Schritt v) auf 7,5-10.

Die Konzentration an Hämoglobin beträgt vorzugsweise 200-380 g/L, insbesondere 240-360 g/L, die Lösung enthält weiterhin 10 bis 150 mmol/L NaHCO₃ sowie 10 bis 150 mmol/L NaCl.

Die Temperatur während der "Eintopf-Reaktion" beträgt 2-42°C, insbesondere 3-25°C, bevorzugt 4-22°C.

Der erfindungsgemäß hergestellte künstliche Sauerstoffträger weist vorzugsweise einen n50-Wert (Kooperativität) von 1,6 bis 2,5 und einen p50-Wert (Halbsättigungsdruck) von 16 bis 24 Torr auf.

Das erfindungsgemäß erhaltene Produkt mit den genannten Charakteristiken kann verwendet werden zur Herstellung eines Mittels zur intravasalen oder biomedizinischen Anwendung als künstlicher Sauerstoffträger, oder in Form einer pharmazeutischen Zubereitung als ein Ersatz des Blutes (Blutsubstitut) oder als ein Zusatz zum Blut zur Erhöhung der Sauerstofftransport-Kapazität (Blutadditiv) oder zu einer Nährlösung, im menschlichen und tierischen Organismus, in einzelnen Organen oder in biotechnischen Anwendungen, insbesondere zur Behandlung eines chronischen Sauerstoffmangels beim Menschen.

Zur Herstellung der zu verabreichenden Produkte werden die erfindungsgemäßen Hämoglobin-Derivate in geeigneten Medien, wie Infusionslösungen, beispielsweise in wässriger Kochsalz- oder Glukoselösung, vorzugsweise in dem Blutplasma isotonischen Konzentrationen, gelöst.

Das erfindungsgemäße Verfahren beruht somit auf einzelnen aufeinander abgestimmten Reaktionsschritten, deren Bedeutung und Auswirkung nachfolgend erläutert wird.

Als Ausgangsmaterial dient sehr reines Hämoglobin. Dieses kann nach bekannten Verfahren aus frischem Blut von Schlachttieren oder beispielsweise aus überalterten Blutkonserven gewonnen werden. Verfahren zur Gewinnung gereinigten Hämoglobins sind beschrieben in: Antonini E. et al. (Hrsg.): Hemoglobin (Colowick S.P., Kaplan N.O. (Hrsg.): Methods in Enzymology, Volume 76), Academic Press, New York u.a. 1981.

Wie erwähnt, ist erfindungsgemäß während der Vernetzung mit Glutardialdehyd, welche an sich bekannt ist, vgl. DE 24 99 885, US 4 857 636, US-A 4 001 200, US-A 4 001 401, DE 4 49 885, US 5 439 882, EP-A 0 201 618, das Hämoglobin desoxygeniert (d. h. von seinem physiologischen Liganden Sauerstoff befreit), denn nur aus während der Vernetzung desoxygeniertem Hämoglobin hergestellte Polymere besitzen Sauerstoffbindungseigenschaften, die einen Einsatz als künstlicher Sauerstoffträger in den gewünschten Indikationen ermöglichen.
Vorzugsweise kann zum weiteren Schutz vor Oxidation des Hämoglobins durch Spuren von verbliebenem Sauerstoff dieser durch chemische Reaktion mit Ascorbat-Ionen entfernt werden.
Als Effektor der Sauerstoffbindung wird insbesondere Pyridoxal-5'-phosphat, in einer an den funktionellen Eigenschaften des Endproduktes optimierten Menge, kovalent an das Schweine- oder Humanhämoglobin angebunden. Diese Anbindung von Pyridoxal-5'-phosphat an Hämoglobin ist prinzipiell bekannt, beispielsweise beschreibt die Patentschrift EP-P 0 528 841 ein Verfahren zur Herstellung pyridoxilierten Hämoglobins. Die Pyridoxilierung führt zu dem gewünschten Halbsättigungsdruck-Werten, wenn so wie erfindungsgemäß beschrieben, vorgegangen wird.
Die Verknüpfungsstellen (Aldimine = Schiffsche Basen) der nicht stabilen kovalenten Anknüpfung von Pyridoxal-5'-phosphat können bekanntlich (s.o.) durch Reduktion mit Natriumborhydrid stabilisiert (es entstehen Amine) werden, wobei erfindungsgemäß die genannten besonderen Bedingungen eingehalten werden. Zum Schutz der Fähigkeit der Hämoglobinmoleküle zu einer homotropen Kooperativität der Sauerstoffbindungsstellen mit einander, die durch die Vernetzung des Hämoglobins mit dem Vernetzer Glutardialdehyd meist deutlich verloren geht, wird erfindungsgemäß vor der Vernetzung 2,3-Bisphosphoglyzerat, ein (heterotroper) chemisch nicht reaktiver Effektor der Sauerstoffbindung des Hämoglobins, zugegeben. Dieser kann somit während der Vernetzung reversibel an seine Bindungsstelle im Hämoglobinmolekül angelagert sein. Nach der Synthese wird 2,3-Bisphosphoglyzerat zusammen mit unverbrauchten Reaktanden sowie überflüssigen Reaktionsprodukten vollständig entfernt (siehe weiter unten).
Als bifunktioneller Vernetzer wird Glutardialdehyd unter den erfindungsgemäßen oben angegebenen Bedingungen eingesetzt.
Die Verknüpfungsstellen (Aldimine = Schiffsche Basen) der vernetzenden molekularen Glutardialdehyd-Brücken werden wie beschrieben durch Reduktion mit Natriumborhydrid stabilisiert (es entstehen Amine), wobei die erfindungsgemäßen Bedingungen einzuhalten sind.
Durch die Vernetzung entstehen vernetzte Hämoglobine mit einer Verteilung der Molekulargewichte zwischen etwa 50 000 und 5 000 000 g/mol (und größer; z.B. 10-15 000 000 g/mol).
Zur Verbesserung der Verträglichkeit mit Plasmaproteinen wird an das vernetzte Hämoglobin ein Polyethylenoxid (MVV)-Derivat, insbesondere dasoben erwähnte monofunktionell aktivierte (Methoxy-) Polyethylenglykol mit einem Molekulargewicht von 1500-2500 g/mol angeknüpft. Die Anknüpfung von Polyethylenglykol (PEG), die sogenannte Pegylierung an als auch die Vernetzung von Hämoglolbinen ist an sich bekannt (vgl. auch E. Tsucheda (Hfsg): Blood Substituts: Present and Future Perspectives, Elsevier Science, Amsterdam 1998).

Neu sind jedoch sowohl Pegylierung als auch Vernetzung von Hämoglobinen zusammen, sowie die Anwendung des chemisch nicht wirksamen Effektors vor der Vernetzung, was insbesondere zum Erhalt der Kooperativität beiträgt, als auch insbesondere die geschilderten Vernetzungsbedingungen. Durch die hier vorgenommene Pegylierung wird nunmehr die ohnehin schwache Reaktion des RES und auch der enzymatische Abbau durch Proteasen verhindert.

Die pH-Wert-Einstellung erfolgt wie oben geschildert.

Durch die erhaltene Reaktionsabfolge und Bedingungen hierfür kann so zum einen die Entstehung einer Perkolationsverteilung verhindert werden (durch die erfindungsgemäße Verdünnung), andererseits können auch die durch Vernetzung und kovalente Anbindungen an Hämoglobin z.B. von Glutardialdehyd, Pyridoxalphosphat und Polyethylenoxid bedingten unerwünschten Änderungen der Sauerstoffaffinität und Kooperativität vermieden werden und insbesondere auch eine hohe Plasmaverträglichkeit erzielt werden.

Alle Reaktionsschritte tragen zusammen zu diesen besonderen Eigenschaften des erfindungsgemäß hergestellten Produktes bei.

Die weitere Aufarbeitung liegt im Rahmen der Kenntnis des Fachmannes: Unlösliche Bestandteile können durch Zentrifugation (beispielsweise für 20 Minuten mit einer relativen Zentrifugalbeschleunigung von 20000 g) abgetrennt werden.
Die vernetzten Hämoglobine werden durch einen (bekannten) präparativen Verfahrensschritt, beispielsweise durch eine Volumenausschluss-Chromatographie oder eine Ultrafiltration, eine fraktionierte Fällung oder eine Feld-Flüss-Fraktionierung, in einen nieder- und einen höher molekularen Anteil aufgetrennt. Bei Wahl geeigneter Methoden (besonders wichtig sind beispielsweise die Nominelle Molekulargewichts-Trenngrenze der Ultrafiltrationsmembrane oder der Molekulargewichts-Trennbereich des verwendeten Gels) werden dabei zugleich alle unverbrauchten Reaktanden sowie unerwünschte Reaktionsprodukte entfernt.

Eine besonders bevorzugte Ausgestaltung der Erfindung ist anhand des nachfolgenden Herstellungsbeispiels näher erläutert:

Gereinigtes Schweine- oder Humanhämoglobin mit einer Konzentration zwischen 200 und 380 g/L, bevorzugt zwischen 240 und 360 g/L, ist in einem wässrigen Elektrolyten gelöst. Dieser enthält Natriumhydrogenkarbonat mit einer Konzentration zwischen 10 und 150 mmol/L, bevorzugt zwischen 40 und 60 mmol/L, sowie Natriumchlorid mit einer Konzentration zwischen 10 und 150 mmol/L, bevorzugt zwischen 50 und 100 mmol/L.
Die Temperatur beträgt von 2 bis 42 °C, bevorzugt zwischen 3 und 25 °C.
Diese Hämoglobinlösung wird gerührt, durch Überströmen reinen Stickstoffs erfolgt eine Desoxygenierung des Hämoglobins.
Zu dieser Lösung werden 2 bis 8, bevorzugt 3 bis 4 mol Natrium-Ascorbat (als 1-molare Lösung in Wasser) pro Mol Hämoglobin zugegeben und zwischen 0,5 und 6 h, bevorzugt zwischen 70 und 120 Minuten, reagieren lassen.
Der pH-Wert der Lösung wird nun mit Milchsäure oder Natronlauge (einer Konzentration zwischen 0,1 und 1, bevorzugt zwischen 0,4 und 0,6 mol/L) auf einen Wert zwischen 6,5 und 7,5, bevorzugt zwischen 6,9 und 7,3 titriert.
Nun werden 0,5 bis 3,0, bevorzugt 1,0 bis 2,5 mol Pyridoxal-5'-Phosphat je Mol Hämoglobin zugegeben und zwischen 0,5 und 20, bevorzugt zwischen 1 und 7 h, reagieren lassen.
Der pH-Wert wird mit Natronlauge oder Milchsäure (einer Konzentration zwischen 0,1 und 1, bevorzugt zwischen 0,4 und 0,6 mol/L) auf einen Wert zwischen 7,5 und 8,5, bevorzugt zwischen 7,7 und 8,2, eingestellt.
Jetzt werden 1,0 bis 9,0, bevorzugt etwa 1,0 bis 2,5 Mol Natriumborhydrid (als 1-molare Lösung in 0,01-molarer Natronlauge) zugegeben und.zwischen 30 und 90, bevorzugt zwischen 50 und 70 Minuten, reagieren lassen.
Der pH-Wert der Lösung wird mit Milchsäure oder Natronlauge (einer Konzentration zwischen 0,1 und 1, bevorzugt zwischen 0,4 und 0,6 mol/L) auf einen Wert zwischen 6,5 und 7,5, bevorzugt zwischen 6,9 und 7,5 titriert.
Nun werden 0,5 bis 6,0, bevorzugt 1,0 bis 4,0 Mol 2,3-Bisphosphoglyzerat je Mol Hämoglobin zugegeben und zwischen 5 und 50, vorzugsweise zwischen 10 und 20 Minuten, reagieren lassen.
Anschließend erfolgt die kontrollierte zeitgesteuerte Zugabe des bifunktionellen Vernetzers, es werden zwischen 6 und 10, bevorzugt zwischen 7 und 9 Mol Glutardialdehyd je Mol Hämoglobin, gelöst in 1-4, bevorzugt in 1,5 bis 2 L Wasser je Liter Hämoglobinlösung, innerhalb 3 bis 10, bevorzugt innerhalb 4 bis 6 Minuten zugegeben und zwischen 1 und 6, bevorzugt zwischen 2 und 3 h, reagieren lassen.

Der pH-Wert wird mit Natronlauge oder Milchsäure (einer Konzentration zwischen 0,1 und 1, bevorzugt zwischen 0,4 und 0,6 mol/L) auf einen Wert zwischen 7,5 und 9,0, bevorzugt zwischen 7,6 und 8,8, eingestellt.
Es werden 5 bis 20, bevorzugt 6 bis 12 Mol Natriumborhydrid (als 1-molare Lösung in 0,01-molarer Natronlauge) je Mol Hämoglobin, zugegeben und zwischen 15 und 100, bevorzugt zwischen 30 und 80 Minuten, reagieren lassen.
Dann erfolgt eine Zugabe von 0 bis 4, bevorzugt zwischen 0,5 und 3 L Wasser je Liter der ursprünglichen Hämoglobiniösung. Der pH-Wert wird, falls erforderlich, mit Natronlauge oder Milchsäure (einer Konzentration zwischen 0,1 und 1, bevorzugt zwischen 0,4 und 0,6 mol/L) auf einen Wert zwischen 7,5 und 10, bevorzugt zwischen 8 und 9, eingestellt.
Nun werden je Mol monomeren Hämoglobins zwischen 2 und 12, bevorzugt zwischen 3 und 8 mol eines aktivierten Polyethylenoxid-Derivat, bevorzugt Methoxy-Succinimidylpropionat-Polyethylenglykol, mit einem Molekulargewicht zwischen 500 und 3000, bevorzugt 1000 und 2500 g/Mol, insbesondere 2000 g/Mol zugegeben.
Anschließend wird, bei weiterem Rühren der Hämoglobinlösung, die Stickstoffatmosphäre für 1 bis 3 Stunden durch reinen Sauerstoff ersetzt und das Hämoglobin so zügig oxygeniert.
Die Aufarbeitung erfolgt wie oben geschildert.

Die Vorteile des erfindungsgemäßen Verfahrens lassen sich somit wie folgt zusammenfassen:

Durch die erfindungsgemäße Reaktionsabfolge, insbesondere die Reaktionsvolumenerhöhung in Schritt iv) des Verfahrens, wird ein Produkt erhalten, welches vollständig zur Herstellung künstlicher Sauerstoffträger verwendet werden kann und zwar je etwa hälftig als Blut-Additiv (die Fraktion mit den vernetzten Hämoglobine höherer Polymerisationsgrade: Fraktion I) und als Blutvolumen-Substitut (Fraktion II, mit den nieder-molekularen Anteilen). Die Trennung kann in einfacher Weise mit bekanntem Verfahren erfolgen, einige mögliche Methoden sind beispielsweise in den Patentschriften EP-A 95 107 280 und EP-A 97 100 790 angeführt. Die Polymeren der Fraktion I, vorzugsweise bis zu einem Molekulargewicht von größer 700 000 g/Mol sind so hinreichend molekular einheitlich, dass sie in der wünschenswerten therapeutischen Plasmakonzentration einen ausreichend geringen kolloidosmotischen Druck besitzen. Durch diese Molekulareinheitlichkeit wird zugleich ein kleiner Virialkoeffizient, wie auch eine geringe Viskosität erreicht. Die Verträglichkeit mit den Proteinen des Blutplasmas, ausreichend große lmmunverträglichkeit und intravasale Verweildauer, wie auch eine ausreichend geringe vasokonstriktorische Nebenwirkungen, d. h. eine geringe Extravasation, der Polymeren der Molekülfraktion I wird durch eine kovalente Anknüpfung von Polyalkylenoxiden erzielt. Darüberhinaus wird der Forderung nach Einfachheit und Wirtschaftlichkeit bei diesem neuen Verfahren in entscheidender Weise insofern Rechnung getragen, als die gesamte Herstellung in einem einzigem Gefäß stattfindet (sogenannte Eintopf-Herstellung) und hohe Ausbeuten von über 70% erzielt werden, wobei die Ausbeute an Polymeren mit einem Molekulargewicht von über 700 000 g/mol mehr als 15 % beträgt.
Das Herstellungsverfahren ermöglicht die Präparation modifizierter und vernetzter Hämoglobine in wenigen Verfahrensschritten. Die gewählten Verfahrensparameter führen dabei zu einer definierten Verteilung modifizierter Hämoglobin-Polymeren, die als künstliche Sauerstoffträger geeignet auch den physiologischen Gegebenheiten im Blutserum Rechnung trägt.

Weiterhin ist die Kooperativität des unvernetzten Hämoglobins im vernetzten Produkt weitgehend erhalten und der Halbsättigungsdruck geeignet justierbar.

Die erfindungsgemäß hergestellten künstlichen Sauerstoffträger aus vernetztem Hämoglobin sind bei parenteraler Applikation plasmaverträglich, und können klinisch wie geschildert angewendet werden.

Die Erfindung wird anhand der folgenden Beispiele näher erläutert.

Dabei zeigen die Figuren 1-3 folgendes:
Figur 1: Eine massen-gewichtete Verteilung der Molekülgrößen und Molekulargewichte (M) des Schweinehämoglobin-Polymeren aus Beispiel 1, dargestellt als Volumenausschluss-Chromatogramm (erhalten mit Sephacryl S-400 HR - Gel, Pharmacia, Freiburg, D). E₄₂₅ₙₘ ist die Extinktion im Chromatographie-Eluat bei 425 nm. Eingezeichnet sind die Abszisssenwerte 700 000 und 5 000 000 g/Mol.
Figur 2: Chromatogramm für Anwendungsbeispiel 2, Erläuterungen vgl. Fig. 1
Figur 3: Chromatogramm für Anwendungbeisiel 3, Erläuterungen vgl. Fig. 1

Ferner wurden folgende Bestimmungsmethoden angewendet:
1. Hämoglobingehalte wurden fotometrisch mit der modifizierten Cyanhämiglobin-Methode nach Drabkin ("Hämoglobin-Farbtest MRP 3", Boehrimger Mannheim, D),
2. pH-Werte wurden potentiometrisch (pH-Glaselektrode) mit einem Blutgasanalysator ("ABL 5", Radiometer, Willich, D) gemessen.
3. Bestimmungen der Molekulargewichtsverteilung der vernetzten Hämoglobine erfolgten durch Volumenausschluss-Chromatografie (gemäß: Pötzschke H. et al. (1996): "Vernetzte globuläre Proteine - eine neue Klasse halbsynthetischer polymerer Moleküle: Charakterisierung ihrer Struktur in Lösung am Beispiel hyperpolymeren Hämoglobins und Myoglobins mittels Volumenausschluss-Chromatographie, Viskosimetrie, Osmometrie und Lichtstreuung", *Macromolecular Chemistry and Physics* 197, 1419 - 1437, sowie Pötzschke H. et al. (1996): "Ein neuartiges Verfahren zur Bestimmung Molarer Massen breit verteilter Polymerer mit Hilfe der Gel-Chromatographie und der Viskosimetrie am Beispiel Hämoglobin-Hyperpolymerer", *Macromolecular Chemistry and Physics* 197, 3229 - 3250) am Gel "Sephacryl S-400 HR" (Pharmacia Biotech, Freiburg, D).
4. Bestimmungen der Charakteristik der Sauerstoffbindung durch Hämoglobine erfolgte mittels einer eigenen Methode und Apparatur (wie beschrieben in: Barnikol W. K. R. et al. (1978): "Eine verbesserte Modifikation der Mikromethode nach Niesel und Thews zur Messung von O₂-Hb-Bindungskurven in Vollblut und konzentrierten Hb-Lösungen", *Respiration* 36, 86 - 95).
5. Die Untersuchung der Plasmaverträglichkeit vernetzter Hämoglobine erfolgte mittels eines standardisierten *in vitro -* Fällungstests (Domack U. (1997), "Entwicklung und *in vivo -* Evaluation eines künstlichen Sauerstoffträgers auf Basis von Rinderhämoglobin", *Dissertation, Fachbereich Chemie, Johannes Gutenberg-Universität,* Mainz 1997): Hämoglobinlösungen (Hämoglobingehalte etwa 30 g/L, in einem wässrigen Elektrolyten (StLg) der Zusammensetzung 125 mM NaCl, 4,5 mM KCI und 3 mM NaN₃) wurden mit gleichen Mengen frisch gewonnenen, steril filtrierten menschlichen Plasmas gemischt und anschließend zu jeweils 500 µL der Mischung bis zu 20 µL 0,5-molare Milchsäure zugesetzt und eingemischt, so dass sich für jedes zu untersuchende Hämoglobin-Derivat jeweils pH-Werte aus einem Bereich zwischen etwa 7,4 bis 6,8 ergaben. Nach einer Inkubation von 30 Minuten bei Raumtemperatur und Zentrifugation der Proben erfolgte die Bestimmung des Hämoglobingehaltes als Maß für das nicht ausgefällte Hämoglobin sowie des zugehörigen pH-Wertes im Überstand, sowie die subjektivoptische Kontrolle auf ungefärbte Ausfällungen von Plasmaproteinen.

### Ausführungsbeispiel 1:

### Herstellung eines erfindungsgemäßen vernetzten und molekular modifizierten Schweinehämoglobins bei 4 °C gemäss dem allgemeinen Herstellungsverfahren

Hochreines Schweinehämoglobin, in einer Konzentration von 330 g/L gelöst in einem wässrigen Elektrolyten der Zusammensetzung 50 mM NaHCO₃ und 100 mM NaCl, wurde bei 4 °C durch Rühren der Lösung unter ständig erneuertem, reinen Stickstoff über der Lösung desoxygeniert. Anschließend wurden 4 mol Natrium-Ascorbat (als 1-molare Lösung in Wasser) pro Mol (monomeren) Hämoglobins zugegeben und 6 h reagieren lassen. Die Lösung wurde mit 0,5-molarer Milchsäure auf einen pH-Wert von 7,1 titriert, 1,1 Mol Pyridoxal-5'-Phosphat je Mol Hämoglobin zugegeben und für 16 h reagieren lassen. Nun wurde mit 0,5-molarer Natronlauge ein pH-Wert von 7,8 eingestellt, 1,1 Mol Natriumborhydrid (als 1-molare Lösung in 0,01-molarer Natronlauge) zugegeben und für eine Stunde reagieren lassen. Jetzt wurde mit 0,5-molarer Milchsäure ein pH von .7,3 eingestellt, zunächst 1,1 Mol 2,3-Bisphosphoglyzerat pro Mol Hämoglobin und nach 15 min Reaktionszeit 8 Mol Glutardialdehyd je Mol Hämoglobin, gelöst in 1,8 L reinem Wasser je Liter Hämoglobinlösung zur Vernetzung des Hämoglobins innerhalb 5 Minuten zugegeben und 2,5 h reagieren lassen. Nach Titration mit 0,5-molarer Natronlauge auf einen pH-Wert von 7,8 folgte eine Zugabe von 15 Mol Natriumborhydrid (als 1-molare Lösung in 0,01-molarer Natronlauge) je Mol Hämoglobin für 1 h. Es erfolgte eine Zugabe von 2 Liter Wasser jeLiter ursprünglicher Hämoglobinlösung. Der pH-Wert betrug dann 9,3, und es folgte direkt eine Zugabe von 4 Mol Methoxy-Succinimidylpropionat-Polyethylenglykol des Molekulargewichts 2000 g/Mol für 2 h. Die Stickstoffatmosphäre über der Lösung wurde durch reinen Sauerstoff ersetzt.
Nach 1 h wurden unlösliche Bestandteile durch Zentrifugation (20000 g für 15 min) abgetrennt. Anschließend erfolgte ein Wechsel des Elektrolyten durch eine Volumenausschluss-Chromatographie (Sephadex G-25 - Gel, Pharmacia, D) zu einer wässrigen Elektrolyt-Lösung der Zusammensetzung 125 mM NaCl, 4,5 mM KCl und 20 mM NaHCO₃.

Die Ausbeute betrug 77 %; die Ausbeute für Molekulargewicht größer 700 000 g/Mol ist 28 %.
Fig. 1 zeigt eine Darstellung der Verteilung der Molaren Massen der erhaltenen Hämoglobin-Polymere in Form eines Volumenausschluss-Chromatogrammes.
Messungen der Charakteristik der Sauerstoffbindung unter physiologischen Bedingungen (eine Temperatur von 37 °C, ein Kohlendioxid-Partialdruck von 40 Torr und ein pH-Wert von 7,4) ergaben für das Produkt einen p50-Wert von 22 Torr und einen n50-Wert von 1,95.
Im "Fällungstest" zeigte das vernetzte Schweinehämoglobin im pH-Bereich von 7,4 bis 6,8 keinerlei Wechselwirkungen mit menschlichem Plasma, insbesondere keine nachweisbaren Fällungen, weder des Hämoglobins, noch von Plasmaproteinen.

### Ausführungsbeispiel 2:

### Herstellung eines erfindungsgemäßen vernetzten und molekular modifizierten Schweinehämoglobins bei Raumtemperatur gemäss dem allgemeinen Herstellungsverfahren

Hochreines Schweinehämoglobin, in einer Konzentration von 330 g/L gelöst in einem wässrigen Elektrolyten der Zusammensetzung 50 mM NaHCO₃ und 100 mM NaCl, wurde bei 22 °C durch Rühren der Lösung unter ständig erneuertem, reinen Stickstoff desoxygeniert. Anschließend wurden 4 mol Natrium-Ascorbat (ais 1-molare Lösung in Wasser) pro Mol (monomeren) Hämoglobins zugegeben und 90 min reagieren lassen. Die Lösung wurde mit 0,5-molarer Milchsäure auf einen pH-Wert von 7,1 titriert, 1,1 mol Pyridoxal-5'-Phosphat je Mol Hämoglobin zugegeben und für 2 h reagieren lassen. Nun wurde mit 0,5-molarer Natronlauge ein pH-Wert von 7,8 eingestellt, 1,5 Mol Natriumborhydrid (als 1-molare Lösung in 0,01-molarer Natronlauge) zugegeben und für eine Stunde reagieren lassen. Jetzt wurde mit 0,5-molarer Milchsäure ein pH von 7,3 eingestellt, zunächst 1,5 mol 2,3-Bisphosphoglyzerat pro Mol Hämoglobin und nach 15 min Reaktionszeit 9 Mol Glutardialdehyd je Mol Hämoglobin, gelöst in 1,8 L reinem Wasser je Liter Hämoglobinlösung zur Vernetzung des Hämoglobins innerhalb 5 Minuten zugegeben und 1 h reagieren lassen. Nach Titration mit 0,5-molarer Natronlauge auf einen pH-Wert von 7,8 folgte eine Zugabe von 10 Mol Natriumborhydrid (als 1-molare Lösung in 0,01-molarer Natronlauge) je Mol Hämoglobin für 0,5 h. Der pH-Wert betrug 8,7 und es folgte direkt eine Zugabe von 8 Mol Methoxy-Succinimidylpropionat-Polyethylenglykol des Molekulargewichts 1000 g/Mol für 1 h. Die Stickstoffatmosphäre wurde durch reinen Sauerstoff ersetzt.
Nach 1 h wurden unlösliche Bestandteile durch Zentrifugation (10 min mit 20000 g) abgetrennt. Anschließend erfolgte ein Wechsel des Elektrolyten durch eine Volumenausschluss-Chromatographie (Sephadex G-25 - Gel, Pharmacia, D) zu einer wässrigen Elektrolyt-Lösung der Zusammensetzung 125 mM NaCl, 4,5 mM KCI und 20 mM NaHCO₃.

Die Ausbeute betrug 79 %; die Ausbeute für Molekulargewichte größer als 700 000 g/Mol betrug 28 %.

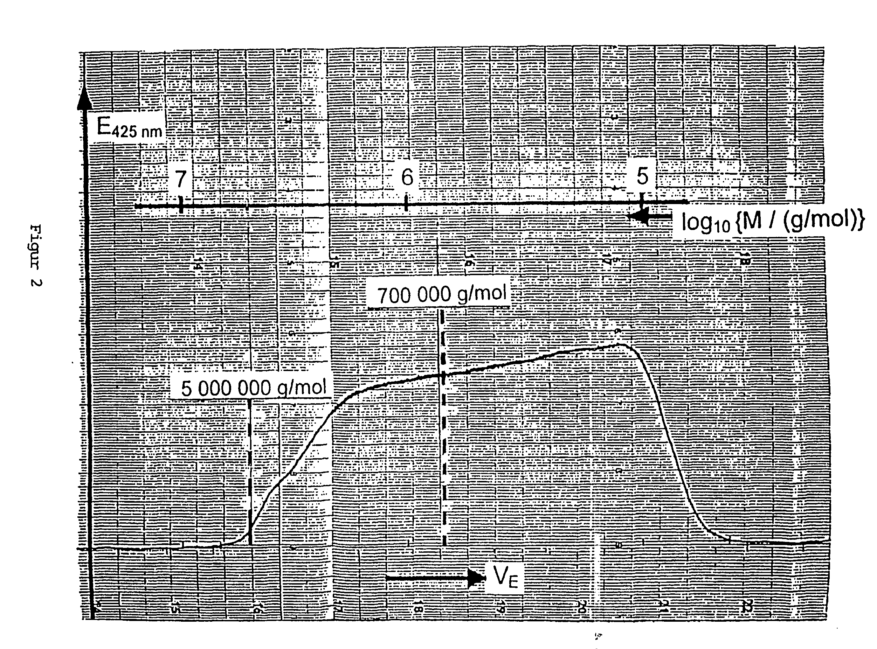

Fig. 2 zeigt eine Darstellung der Verteilung der Molaren Massen der erhaltenen Hämoglobin-Polymere in Form eines Volumenausschluss-Chromatogrammes. Messungen der Charakteristik der Sauerstoffbindung unter physiologischen Bedingungen (eine Temperatur von 37°C, ein Kohlendioxid-Partialdruck von 40 Torr und ein pH-Wert von 7,4) ergaben für das Produkt einen p50-Wert von 22 Torr und einen n50-Wert von 1,6.
Im "Fällungstest" zeigte das vernetzte Schweinehämoglobin im physiologisch und pathophysiologisch interessanten pH-Bereich von 7,4 bis 6,8 keinerlei Wechselwirkungen mit menschlichem Plasma, insbesondere keine nachweisbaren Fällungen, weder des Hämoglobins, noch von Plasmaproteinen.

### Anwendungsbeispiel 3:

### Herstellung eines erfindungsgemäßen vernetzten und molekular modifizierten menschlichen Hämoglobins (bei 4°C) gemäss dem allgemeinen Herstellungsverfahren

Hochreines Humanhämoglobin, in einer Konzentration von 330 g/L gelöst in einem wässrigen Elektrolyten der Zusammensetzung 50 mM NaHCO₃ und 100 mM NaCl, wurde bei 4 °C durch Rühren der Lösung unter ständig erneuertem, reinen Stickstoff desoxygeniert. Anschließend wurden 4 mol Natrium-Ascorbat (als 1-molare Lösung in Wasser) pro Mol (monomeren) Hämoglobins zugegeben und 3 h reagieren lassen. Die Lösung wurde mit 0,5-molarer Milchsäure auf einen pH-Wert von 7,1 titriert, 1,1 mol Pyridoxal-5'-Phosphat je Mol Hämoglobin zugegeben und für 16 h reagieren lassen. Nun wurde mit 0,5-molarer Natronlauge ein pH-Wert von 7,8 eingestellt, 1,5 Mol Natriumborhydrid (als 1-molare Lösung in 0,01-molarer Natronlauge) zugegeben und für eine Stunde reagieren lassen. Jetzt wurde mit 0,5-molarer Milchsäure ein pH von 7,3 eingestellt, zunächst 1,5 mol 2,3-Bisphosphoglyzerat pro Mol Hämoglobin und nach 1-5 min Reaktionszeit 9 mol Glutardialdehyd je Mol Hämoglobin, gelöst in 1,8 L reinem Wasser je Liter Hämoglobinlösung zur Vernetzung des Hämoglobins innerhalb 5 Minuten gleichmäßig zugegeben und 2,5 h reagieren lassen. Nach Titration mit 0,5-molarer Natronlauge auf einen pH-Wert von 8,0 folgte eine Zugabe von 10 Mol Natriumborhydrid (als 1-molare Lösung in 0,01-molarer Natronlauge) je Mol Hämoglobin für 1 h.Dann erfolgt eine Zugabe von Wasser, 2 L pro Liter ursprüngliche Hämoglobinlösung. Der pH-Wert betrug dann 8,6 und es folgte direkt eine Zugabe von 4 Mol Methoxy-Succinimidylpropionat-Polyethylenglykol des Molekulargewichts 2000 g/mol für 2 h. Die Stickstoffatmosphäre wurde durch reinen Sauerstoff ersetzt. Nach 1 h wurden unlösliche Bestandteile durch Zentrifugation (10 min mit 20000 g) abgetrennt. Anschließend erfolgte ein Wechsel des Elektrolyten durch eine Volumenausschluss-Chromatographie (Sephadex G-25 - Gel, Pharmacia, D) zu einer.wässrigen Elektrolyt-Lösung der Zusammensetzung 125 mM NaCl, 4,5 mM KCI und 20 mM NaHCO₃.

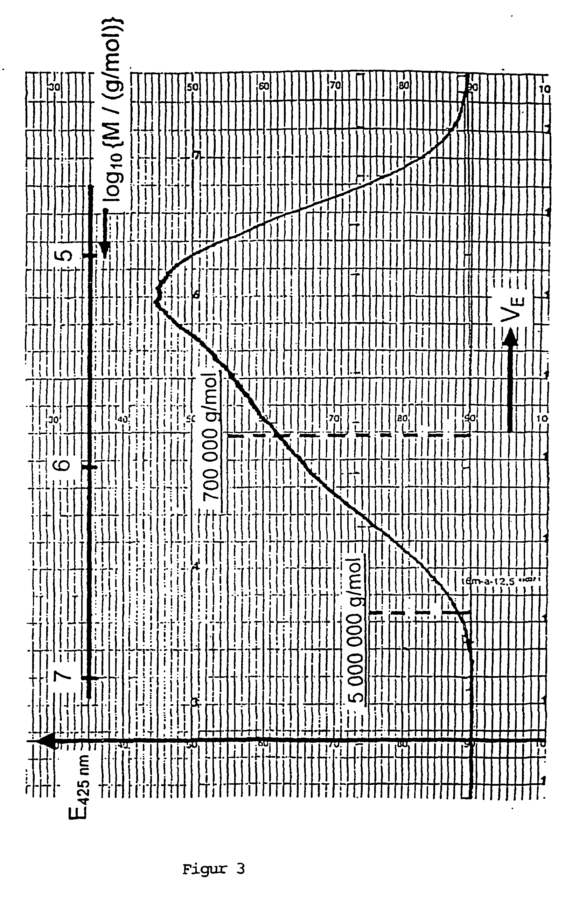

Fig. 3 zeigt eine Darstellung der Verteilung der Molaren Massen der erhaltenen Hämoglobin-Polymere in Form eines Volumenausschluss-Chromatogrammes.
Die Gesamtausbeute betrug 75 %; die Ausbeute an Polymerem mit einem Molekulargewicht größer als 700 000 g/Mol betrug 17 %.
Messungen der Charakteristik der Sauerstoffbindung unter physiologischen Bedingungen (eine Temperatur von 37 °C, ein Kohlendioxid-Partialdruck von 40 Torr und ein pH-Wert von 7,4) ergaben für das Produkt einen p50-Wert (als Maß einer mittleren Sauerstoffaffinität) von 21 Torr und einen n50-Wert (eine mittlere scheinbare Kooperativität der Sauerstoffbindungsstellen) von 1,74.
Im "Fällungstest" zeigte das vernetzte Schweinehämoglobin im physiologisch und pathophysiologisch interessanten pH-Bereich von 7,4 bis 6,8 keinerlei Wechselwirkungen mit den Proteinen menschlichen Plasmas, insbesondere keine nachweisbaren Fällungen, weder des Hämoglobins, noch von Plasmaproteinen.

## Patentansprüche

1. Verfahren zur Herstellung künstlicher Sauerstoffträger aus vernetztem Hämoglobin mit verbesserten funktionellen Eigenschaften, **dadurch gekennzeichnet,**
**dass** Hämoglobin
i) zunächst desoxygeniert wird;
ii) anschließend kovalent mit einem chemisch reaktiven Effektor der Sauerstoffbindung umgesetzt wird;
iii) dann die Lösung mit einem nicht chemisch reaktiven Effektor versetzt wird; und sodann
iv) das Hämoglobin mit Glutardialdehyd, unter sehr starker Verdünnung des Reaktionsgemisches bei gleichzeitiger Zugabe des Vernetzers stabil kovalent miteinander vernetzt wird, anschließend die Lösung erneut mit Wasser verdünnt wird, wobei das Volumen des Reaktionsgemisches insgesamt um einen Faktor 2 bis 10 zunimmt und sodann
v) ein Polyethylenoxid kovalent angeknüpft wird,
vi) das erhaltene Produkt in bekannter Weise aufgearbeitet wird.

2. Verfahren nach Anspruch 1, **dadurch gekennzeichnet, dass** das Hämoglobin vom Schwein oder vom Menschen stammt.

3. Verfahren nach Anspruch 2, **dadurch gekennzeichnet, dass** Schweinehämoglobin als Ausgangsmaterial dient.

4. Verfahren nach einem der Ansprüche 1 bis 3, **dadurch gekennzeichnet, dass** in Schritt iv) Glutardialdehyd in einer sehr stark verdünnten Lösung zeitgesteuert zugegeben wird und so das Volumen des Reaktionsgemisches und die Hämoglobinkonzentration während der Polymerisationsreaktion gleichzeitig gegensinnig variiert werden, und anschließend die Lösung verdünnt wird.

5. Verfahren nach Anspruch 4, **dadurch gekennzeichnet, dass** in Schritt iv) Glutardialdehyd in einer Menge von 6-10 Mol, bezogen auf monomeres Hämoglobin, gelöst in 1-2 L Wasser je Liter ursprünglicher Reaktionslösung, innerhalb von 3-15 Minuten zugesetzt wird und weitere 1-6 Stunden reagiert.

6. Verfahren nach einem der Ansprüche 1 bis 5, **dadurch gekennzeichnet, dass** der das Hämoglobin enthaltenden Lösung vor der Umsetzung gemäß dem Schritt ii) 2-8 Mol Natriumsascorbat pro Mol unvernetztes Hämoglobin für 0,5-6 Stunden zugegeben wird.

7. Verfahren nach einem der Ansprüche 1 bis 6, **dadurch gekennzeichnet, dass** in Schritt ii) als Effektor Pyridoxal-5'-phosphat in einem molaren Verhältnis, bezogen auf monomeres Hämoglobin, von 0,5 bis 3, bevorzugt 1 bis 2,5 mol/mol innerhalb von 0,5 bis 20 Stunden kovalent angebunden wird.

8. Verfahren nach Anspruch 7, **dadurch gekennzeichnet, dass** in Schritt ii) sowie in Schritt iv) nach der kovalenten Anbindung von Pyridoxal-5'-phosphat an Hämoglobin sowie nach kovalenter Verknüpfung des Hämoglobins mit Glutardialdehyd jeweils reduktives Natriumborhydrid hinzugesetzt wird.

9. Verfahren nach einem der Ansprüche 1 bis 8, **dadurch gekennzeichnet, dass** in Schritt iii) 2,3-Bisphosphoglyzerat in einer relativen Menge von 0,5-6 Mol, bezogen auf monomeres Hämoglobin, zugesetzt wird und 5-50 Minuten danach Schritt iv) eingeleitet wird.

10. Verfahren nach einem der Ansprüche 1 bis 9, **dadurch gekennzeichnet, dass** in Schritt v) ein Polyethylenglykol-Ester mit einem Molekulargewicht von 500 bis 3000 g/Mol angeknüpft wird

11. Verfahren gemäß einem der Ansprüche 1-10, **dadurch gekennzeichnet, dass** das erhaltene Produkt durch ein präparatives Trennverfahren in eine Fraktion mit großer mittlerer molarer und eine Fraktion mit niederer mittlerer molekularer Masse getrennt wird.

12. Verfahren nach Anspruch 11, **dadurch gekennzeichnet, dass** aus dem niedermolekularen Anteil der Polymeren ein parenterales Blutsubstitut hergestellt wird und aus dem höhermolekularen Anteil der Polymeren ein parenterales Blutadditiv hergestellt wird.

13. Künstlicher Sauerstoffträger, **dadurch gekennzeichnet, dass** es sich um ein mit einem Effektor der Sauerstoffbindung kovalent konjugiertes sowie mit Glutardialdehyd polymerisiertes und mit einem Polyalkylenoxid- Derivat chemisch verknüpftes Hämoglobin handelt.

14. Künstlicher Sauerstoffträger gemäss Anspruch 13, **dadurch gekennzeichnet, dass** der Träger einen n50-Wert von 1,6 bis 2,5 und einen p50-Wert von 16 bis 24 Torr aufweist.

15. Verwendung von vernetztem Hämoglobin gemäss Anspruch 13 oder 14, zur Herstellung eines Mittels zur intravasalen oder biomedizinischen Anwendung als künstlicher Sauerstoffträger im menschlichen oder tierischen Organismus zur Behandlung von Sauerstoffmangelzuständen

16. Verwendung gemäss Anspruch 15 zur Behandlung eines chronischen Sauerstoffmangels beim Menschen.

17. Verwendung gemäß Anspruch 16, **dadurch gekennzeichnet, dass** das Mittel in Form einer pharmazeutischen Zubereitung als ein Ersatz des Blutes (Blutsubstitut) oder als ein Zusatz zum Blut zur Erhöhung der Sauerstofftransport-Kapazität (Blutadditiv) oder in einzelnen Organen verwendet wird.

18. Ex vivo-Verwendung von vernetztem Hämoglobin gemäss Anspruch 13 oder 14 als Zusatz zu einer Nährlösung, oder in biotechnischen Anwendungen.

## Claims

1. Process for the preparation of artificial oxygen carriers made from crosslinked haemoglobin having improved functional properties, **characterised in that** haemoglobin
i) is first deoxygenated;
ii) is then reacted covalently with a chemically reactive effector of the oxygen bond;
iii) then a chemically non-reactive effector is added to the solution; and then
iv) the haemoglobin is crosslinked stably and covalently with glutardialdehyde, with very great dilution of the reaction mixture, with simultaneous addition of the crosslinker, and the solution is subsequently diluted with water again, the volume of the reaction mixture increasing by a factor of from 2 to 10 in total, and then
v) a polyethylene oxide is linked covalently,
vi) the resulting product is worked up in a known manner.

2. Process according to claim 1, **characterised in that** the haemoglobin comes from pigs or from humans.

3. Process according to claim 2, **characterised in that** porcine haemoglobin is used as the starting material.

4. Process according to any one of claims 1 to 3, **characterised in that** in step iv) glutardialdehyde is added in a very highly dilute solution in a time-controlled manner, and the volume of the reaction mixture and the haemoglobin concentration are thus simultaneously varied in opposite directions during the polymerisation reaction, and the solution is subsequently diluted.

5. Process according to claim 4, **characterised in that** in step iv) glutardialdehyde, dissolved in from 1 to 2 litres of water per litre of original reaction solution, is added in an amount of from 6 to 10 mol, based on monomeric haemoglobin, in the course of from 3 to 15 minutes and reacted for a further 1 to 6 hours.

6. Process according to any one of claims 1 to 5, **characterised in that** from 2 to 8 mol of sodium ascorbate per mole of uncrosslinked haemoglobin are added to the solution containing the haemoglobin for a period of from 0.5 to 6 hours before the reaction according to step ii).

7. Process according to any one of claims 1 to 6, **characterised in that** in step ii) pyridoxal-5'-phosphate in a molar ratio, based on monomeric haemoglobin, of from 0.5 to 3 mol/mol, preferably from 1 to 2.5 mol/mol, is bonded covalently as the effector in the course of from 0.5 to 20 hours.

8. Process according to claim 7, **characterised in that** in step ii) and in step iv), after the covalent bonding of pyridoxal-5'-phosphate to haemoglobin and after the covalent linking of the haemoglobin to glutardialdehyde, reductive sodium borohydride is added in each case.

9. Process according to any one of claims 1 to 8, **characterised in that** in step iii) 2,3-bisphosphoglycerate is added in a relative amount of from 0.5 to 6 mol, based on monomeric haemoglobin, and step iv) is initiated from 5 to 50 minutes later.

10. Process according to any one of claims 1 to 9, **characterised in that** in step v) a polyethylene glycol ester having a molecular weight of from 500 to 3000 g/mol is linked.

11. Process according to any one of claims 1 to 10, **characterised in that** the resulting product is separated by a preparative separation method into a fraction have a high mean molar mass and a fraction having a lower mean molecular mass.

12. Process according to claim 11, **characterised in that** a parenteral blood substitute is prepared from the low molecular weight portion of the polymer, and a parenteral blood additive is prepared from the higher molecular weight portion of the polymer.

13. Artificial oxygen carrier, **characterised in that** it is a haemoglobin covalently conjugated with an effector of the oxygen bond as well as polymerised with glutardialdehyde and linked chemically to a polyalkylene oxide derivative.

14. Artificial oxygen carrier according to claim 13, **characterised in that** the carrier has a n50 value of from 1.6 to 2.5 and a p50 value of from 16 to 24 torr.

15. Use of crosslinked haemoglobin according to claim 13 or 14 in the preparation of an agent for intravasal or biomedical application as an artificial oxygen carrier in the human or animal organism for treating conditions of oxygen deficiency.

16. Use according to claim 15 in the treatment of a chronic oxygen deficiency in humans.

17. Use according to claim 16, **characterised in that** the agent is used in the form of a pharmaceutical preparation as a replacement for blood (blood substitute) or as an additive to the blood for increasing the oxygen transport capacity (blood additive) or in individual organs.

18. *Ex vivo* use of crosslinked haemoglobin according to claim 13 or 14 as an additive to a nutrient solution or in biotechnical applications.

## Revendications

1. Procédé de préparation d'un transporteur d'oxygène artificiel à base d' hémoglobine réticulée avec des propriétés fonctionnelles améliorées, **caractérisé en ce que** l'hémoglobine
i) est d'abord désoxygénée ;
ii) est ensuite transformée par covalence avec un effecteur de la liaison de l'oxygène chimiquement réactif ;
iii) ensuite, la solution est additionnée d'un effecteur chimiquement non réactif et ensuite
iv) l'hémoglobine est réticulée ensemble de façon stable avec du glutardialdéhyde moyennant une forte dilution du mélange réactionnel avec une addition simultanée du réticulant, la solution étant ensuite diluée de nouveau avec de l'eau, lors de quoi le volume du mélange réactionnel augmente en tout d'un facteur 2 à 10 et ensuite
v) un poly(oxyde d'éthylène) est greffé dessus de façon covalente,
vi) le produit obtenu est traité de manière connue.

2. Procédé selon la revendication 1, **caractérisé en ce que** l'hémoglobine provient du porc ou de l'homme.

3. Procédé selon la revendication 2, **caractérisé en ce que** de l'hémoglobine porcine sert de produit de départ.

4. Procédé selon l'une des revendications 1 à 3, **caractérisé en ce que** dans l'étape iv) le glutardialdéhyde est ajouté en une solution très fortement diluée de manière commandée par le temps et qu'ainsi le volume du mélange réactionnel et la concentration de l'hémoglobine pendant la réaction de polymérisation varient en même temps en sens inverse, et **en ce que** la solution est ensuite diluée.

5. Procédé selon la revendication 4, **caractérisé en ce que** dans l'étape iv), le glutardialdéhyde est ajouté en l'espace de 3 à 15 minutes en une quantité de 6 à 10 moles, rapportée à l'hémoglobine monomère, dissous dans 1 à 2 L d'eau par litre de la solution de réaction initiale, et qu'on le laisse réagir encore 1 à 6 heures.

6. Procédé selon l'une des revendications 1 à 5, **caractérisé en ce que** l'on ajoute à la solution contenant l'hémoglobine, avant la réaction selon l'étape ii), 2 à 8 moles d'ascorbate de sodium par mole d'hémoglobine non réticulée pendant 0,5 à 6 heures.

7. Procédé selon l'une des revendications 1 à 6, **caractérisé en ce que** dans l'étape ii), comme effecteur, du pyridoxal-5'-phosphate est greffé par covalence dans un rapport molaire, rapporté à l'hémoglobine monomère, de 0,5 à 3, de préférence de 1 à 2,5 mol/mol en l'espace de 0,5 à 20 heures.

8. Procédé selon la revendication 7, **caractérisé en ce que** dans l'étape ii) ainsi que dans l'étape iv), après la greffe covalente de pyridoxal-5'-phosphate sur l'hémoglobine ainsi qu'après l'enchaînement covalent de l'hémoglobine avec le glutardialdéhyde, de l'hydrure de bore-sodium réducteur est ajouté respectivement.

9. Procédé selon l'une des revendications 1 à 8, **caractérisé en ce que** dans l'étape iii), du 2,3-bisphosphoglycérate est ajouté en une quantité relative de 0,5 à 6 mol, rapportée à l'hémoglobine monomère, et **en ce que** l'étape iv) est déclenchée 5 à 50 minutes après.

10. Procédé selon l'une des revendications 1 à 9, **caractérisé en ce que** dans l'étape v), un ester de polyéthylène glycol avec un poids moléculaire de 500 à 3000 g/mol est greffé.

11. Procédé selon l'une des revendications 1 à 10, **caractérisé en ce que** le produit obtenu est séparé par un procédé de séparation préparative en une fraction avec une masse moléculaire moyenne grande et en une fraction avec une masse moléculaire moyenne basse.

12. Procédé selon la revendication 11, **caractérisé en ce qu'**un substitut de sang parentéral est préparé à partir de la part de polymères de bas poids moléculaire, et un additif de sang parentéral est préparé à partir de la part de polymères de poids moléculaire plus élevé.

13. Transporteur d'oxygène artificiel, **caractérisé en ce qu'**il s'agit d'une hémoglobine conjuguée de façon covalente avec un effecteur de la liaison de l'oxygène ainsi que polymérisée avec du glutardialdéhyde et greffée avec un dérivé de poly(oxyde d'alkylène).

14. Transporteur d'oxygène artificiel selon la revendication 13, **caractérisé en ce que** le transporteur présente une valeur n50 de 1,6 à 2,5 et une valeur p50 de 16 à 24 Torr.

15. Utilisation d'une hémoglobine réticulée conformément à la revendication 13 ou 14 à la préparation d'un agent pour une application intravasculaire ou biomédicale comme transporteur artificiel d'oxygène dans l'organisme humain ou animal pour le traitement d'états d'insuffisance d'oxygène.

16. Utilisation conformément à la revendication 15 pour le traitement d'une insuffisance chronique d'oxygène chez l'homme.

17. Utilisation conformément à la revendication 16, **caractérisée en ce que** l'agent est utilisé sous la forme d'une préparation pharmaceutique comme un remplacement du sang (substitut de sang) ou comme une addition à du sang pour l'augmentation de la capacité de transport de l'oxygène (additif du sang), ou dans certains organes.

18. Utilisation ex-vivo d'hémoglobine réticulée selon la revendication 13 ou 14 comme additif à une solution nutritive ou dans des applications biotechniques.
